Europäisches Patentamt

European Patent Office

Office européen des brevets

(19)

(11) Veröffentlichungsnummer: **0 014 316**
**B1**

(12) # EUROPÄISCHE PATENTSCHRIFT

(45) Veröffentlichungstag der Patentschrift:
09.11.83

(51) Int. Cl.³: **A 61 N 1/32**

(21) Anmeldenummer: **80100066.2**

(22) Anmeldetag: **07.01.80**

(54) **Gerät zur Interferenzstromtherapie.**

(30) Priorität: **09.01.79 DE 2900612**

(43) Veröffentlichungstag der Anmeldung:
**20.08.80 Patentblatt 80/17**

(45) Bekanntmachung des Hinweises auf die Patenterteilung:
**09.11.83 Patentblatt 83/45**

(84) Benannte Vertragsstaaten:
**CH FR GB IT NL SE**

(56) Entgegenhaltungen:
**AT - A - 335 049**
**DE - A - 1 214 269**
**DE - A - 2 632 700**
**DE - B - 1 298 557**
**GB - A - 1 502 432**
**US - A - 3 746 891**

(73) Patentinhaber: **Rodler, Hans, Bahnhofplatz 3,
D-8228 Freilassing (DE)**

(72) Erfinder: **Rodler, Hans, Bahnhofplatz 3,
D-8228 Freilassing (DE)**

Gerät zur Interferenzstromtherapie

Die Erfindung betrifft ein Gerät zur Elektrotherapie entsprechend dem Oberbegriff des Anspruches 1.

Bei derartigen Geräten werden zwei unabhängige niederfrequente Wechselströme mittels Elektroden dem Körper zugeführt und in ihm zur Interferenz gebracht. Um therapeutisch wirkungsvoll zu sein, muß die Interferenzschwingung von 0,01–100 Hz einstellbar sein. Außerdem ist es therapeutisch von Vorteil, wenn die Kurvenform dieser Interferenzschwingung je nach Verwendungszweck verändert werden kann, da es bei gewissen Erkrankungsformen von Vorteil ist, wenn zwischen den Interferenzamplituden Pausen liegen, während der die Interferenzhüllkurve Null ist. Auch der ansteigende und der abfallende Teil der Interferenzhüllkurve sollte steuerbar sein, so daß einerseits Rechteck- oder Dreieckinterferenzkurven, sowie andere Kurvenformen erstellt werden können.

Es ist aus der DE-AS 2 143 562 (Rodler) und aus der AT-A-335 049 (Rodler) bekannt, zur Erfüllung dieser Erfordernisse, die durch den Körper fließenden Teilströme durch eine Modulationseinrichtung in ihrer Phase rhythmisch zu verschieben. Bei der rhythmischen Phasenverschiebung hängt die Phasenverschiebung von der Amplitude der Modulationsspannung ab, weshalb sowohl im Bereich der Phasenverschiebung Null als auch im Bereich der Phasenverschiebung von 180 Grad aufwendige Abgleicharbeiten und amplitudenstabilisierte Modulationsspannungen erforderlich sind, um Verzerrungen der Interferenzhüllkurve zu vermeiden. Die Hüllkurvenform muß außerdem durch einen Funktionsgenerator erzeugt werden, der aus vorgenannten Gründen sehr konstante Amplituden liefern muß und dadurch einen kostspieligen Aufbau verursacht. Außerdem ist aus der DE-AS 2 632 700 (Dölker) und aus der DE-AS 1 109 200 (Nemec) bekannt, zwei Teilströme mit geringem Frequenzunterschied zu verwenden, so daß durch den Frequenzunterschied eine fortlaufende Phasenverschiebung zwischen den beiden Teilströmen entsteht und damit ebenfalls eine Interferenzschwingung in der Tiefe eines Körpers auftritt. Hierbei sind nur sinoide Interferenzhüllkurven möglich, die gegenseitig um 180 Grad verschoben überlagert sind, so daß nur ein ganz kurzer Nulldurchgang entsteht, während die Kurvenform der Interferenzhüllkurve nicht beeinflußbar ist.

Der Erfindung liegt die Aufgabe zugrunde, eine Anordnung zu schaffen, die in der Lage ist, ohne den Nachteil der rhythmischen Phasenverschiebung Interferenzkurve der verschiedensten Kurvenform im Überlagerungsgebiet der mit konstanten Amplituden zugeführten Teilströme im behandelten Körper zu erzeugen, mit dieser Anordnung eine Verbesserung der therapeutischen Wirkung zu erzielen und die Anordnung betriebssicherer und wirtschaftlicher zu machen.

Diese Aufgabe wird durch den kennzeichnenden Teil des Anspruchs 1 gelöst. Hierbei ist im Oszillator-Frequenzteiler-Kreis zumindest einem Frequenzteiler eine digitale Auswahleinrichtung angeschlossen, die einzelne Perioden zumindest eines Patientenstromes auswählt und in der Periodendauer verändert, während die dazwischen liegenden Perioden dieses Stromes unverändert bleiben. Die digitale Auswahleinrichtung ist hierbei mit einer Steuereinrichtung verbunden, die die Lage der ausgewählten Perioden festlegt und damit die Phasenlage dieses Patientenstromes im Sinne einer abwechselnd fortlaufenden und abwechselnd gleichbleibenden Phasenverschiebung verändert, wobei die Dauer der gleichbleibenden Phasenverschiebung die Dauer der einzelnen Stufen der Interferenzhüllkurve, die Größe der Änderung der geänderten Periode die Stufenhöhe der Interferenzhüllkurve bestimmt.

Bei dieser erfindungsgemäßen Anordnung besteht daher zumindest ein Patientenstrom abwechseind aus gleichen Periodenlängen wie die übrigen Patientenströme, während zwischendurch kurzzeitig auf digitalem Wege die Periodendauer verändert wird. Diese Veränderung der Periodendauer bewirkt eine digitale Phasenverschiebung zu den anderen Strömen, die lediglich durch den Frequenzunterschied dieser einzelnen Periode gegenüber den anderen Perioden festgelegt ist. Diese geänderte Phasenlage bleibt auch während der wieder gleich langen übrigen Perioden des Stromes erhalten, wodurch ein stufenförmiges digitales Ansteigen der Phasenverschiebung im Verhältnis zu den anderen Strömen erreicht wird. Je stärker die Periodendauer verändert wird, umso höher ist die Stufe der Phasenverschiebung. Es ist also möglich, durch Veränderung der Höhe der Stufe und durch Veränderung der Zeitdauer der bestehen bleibenden Gleichfrequenz verschiedene Kurvenformen zu reproduzieren.

Um die ausgewählte Periodendauer zu verlängern, ist in einer weiteren Ausbildung der Erfindung zwischen Oszillator und Frequenzteiler als digitale Auswahlschaltung eine Takt-Sperrschaltung vorgesehen, die von der Steuerschaltung gesteuert wird und im Bereich der ausgewählten Ausgangsperiode eine vorgegebene Anzahl Taktimpulse sperrt und damit die Periodendauer der ausgewählten Periode verlängert, die Anzahl der ausgesperrten Taktimpulse bestimmt die Größe der Periodenänderung der ausgewählten Ausgangsperiode. Bei einer 15fachen Frequenzteilung ist daher die Verlängerung der Ausgangsperiode, wenn ein Taktimpuls ausgesperrt wird, $1/15$ der Periodendauer.

Um auf einfache Weise fortlaufende Dreieck- oder Rechteck-Interferenzhüllkurven produzieren zu können, wird nach einer weiteren Ausbildung der Erfindung dem Patientenfrequenzausgang ein weiterer Teiler als Steuerschaltung nachgeschaltet, die zur Änderung der Zeitdauer der Stufen mit einer Umschalteinrichtung versehen ist und außerdem eine Umschalteinrichtung zur Veränderung der Ausgangsimpulsbreite besitzt, mit Hilfe derer die Höhe der Stufen verändert werden kann. Wird mit Hilfe der Impulsbreitenumschaltung die Impulsbreite soweit erhöht, daß die Phasenlage innerhalb einer Periode um 180 Grad verschoben wird, dann entstehen als Interferenzhüllkurven Rechteckkurven. Durch Zusammenwirken der beiden Umschalteinrichtungen, bedingt durch automatische Steuerung derselben, kann auf digitalem Wege jede beliebige Kurvenform erzielt werden.

Um die ausgewählte Periodendauer zu verkürzen, ist in einer weiteren Ausbildung der Erfindung zwischen Oszillator und Frequenzteiler die digitale Auswahleinrichtung eine Additionsschaltung, die mit ihrem Ausgang an den Frequenzteiler, mit ihrem einen Eingang über einen Taktteiler an den Oszillator und mit ihrem zweiten Eingang über eine Sperrschaltung mit einem höherfrequenten Ausgang des Taktteilers oder umschaltbar direkt am Oszillator angeschlossen ist, wobei der Schalteingang der Sperrschaltung über ein Undgatter mit dem Patientenfrequenzausgang und der Steuereinrichtung verbunden ist, so daß im Abstand der durch die Steuerschaltung bestimmten Zeit mit der am Patientenfrequenzausgang auftretenden Impulsbreite höherfrequente Taktimpulse den Frequenzteiler erreichen und damit die Periode verkürzen.

Um periodisch an- und abschwellende Interferenzfrequenzen zu erhalten, besteht in einer weiteren Ausbildung der Erfindung die Steuerschaltung aus einem Multiplexer und einem Frequenzteiler, wobei der Multiplexer verschiedene Teilerstufen des Frequenzteilers hintereinander an die digitale Auswahleinrichtung anschließt, der Schalteingang des Multiplexers ist mit einem weiten untersetzten Ausgang des Teilers verbunden, der den Multiplexer schrittweise weiterschaltet.

Da alle zur Realisierung des Erfindungsgedankens notwendigen Bauteile in Mikroprozessoren vereinigt sind, besteht in einer weiteren Ausbildung der Erfindung der Oszillator, die Frequenzteiler, die digitale Auswahleinrichtung und die Steuereinrichtung aus einem Mikroprozessor.

Die mit der Erfindung erzielten Vorteile bestehen insbesondere darin, daß auf digitalem Wege Interferenzkurven verschiedenster Kurvenform mit einfachen Mitteln und handelsüblichen integrierten Bauteilen, wobei die Frequenzkonstanz der Oszillatoren keine Rolle spielt, erzeugt werden. Dreieck- und Rechteckinterferenzkurven können durch einfaches Takten ohne komplizierte Funktionsgeneratoren in beliebiger Zeitausdehnung erzeugt werden. Es können Zeitschaltungen in einfacher Weise unter Ausnützung der Frequenzteiler und der Taktimpulse zur Programmierung von Behandlungszeiten realisiert werden. Die Bedienung kann durch weitgehende Automatisierung wesentlich vereinfacht werden.

Ein besonderer Vorteil besteht darin, daß die Teiler zusammen mit den Multiplexern als Speicherelemente aufgefaßt werden können, wobei mit Hilfe der Verdrahtung verschiedenste Programme realisiert werden können.

Der Vorteil bei Verwendung von Mikroprozessoren ist besonders durch die Programmierbarkeit gegeben, sodaß mit der Software verschiedenste Kurvenformen, Zeitabläufe und Funktionen verwirklicht werden können.

Die Ausführungsbeispiele der Erfindung sind in einer Zeichnung dargestellt und werden im folgenden näher beschrieben.

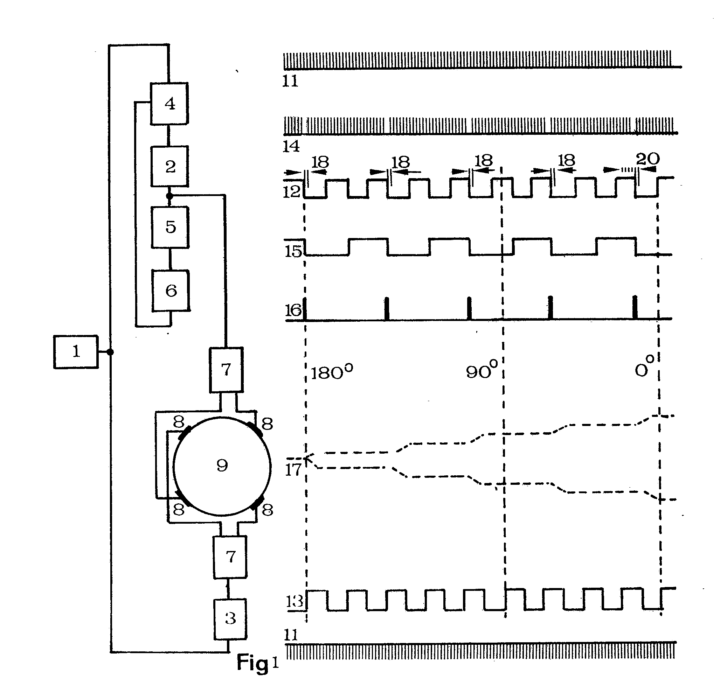

Es zeigt Fig. 1 eine Anordnung zur digitalen Verlängerung der ausgewählten Perioden,

Fig. 2 zeigt eine Anordnung zur digitalen Verkürzung der ausgewählten Perioden und

Fig. 3 zeigt den Kurvenverlauf.

Fig. 1

Ein Oszillator 1 mit einer Frequenz von z. B. 48 KHz erzeugt Taktimpulse entsprechend Kurvenzug 11. Diese Impulse werden einerseits dem Frequenzteiler 3 zugeführt, der durch z. B. 12fache Teilung den Kurvenzug 13 mit 4 KHz erzeugt, die durch die Kurvenumwandlungs- und Verstärkerstufe 7 in eine Sinuskurve umgewandelt werden. Mittels Elektroden 8 wird dieser Sinusstrom dem Körper 9 zugeführt. Andererseits wird die Impulsfrequenz 11 von 48 KHz der digitalen Auswahleinrichtung 4 zugeführt, die aus einem Nandgatter bestehen kann, aber auch durch einen Start-Stopp-Oszillator, der vom Oszillator 1 synchronisiert wird, gebildet werden kann, zugeführt. Am Ausgang der digitalen Auswahleinrichtung 4 entsteht eine Impulskurve 14, bei der einzelne oder mehrere Impulse fehlen. Der Frequenzteiler 2 mit demselben Teilungsverhältnis wie der Frequenzteiler 3, z. B. 12, ergibt am Ausgang während 12 Impulsen die gleiche Periodenlänge wie der Frequenzteiler 3, wobei sich während der fehlenden Takt-Impulse die Periodendauer z. B. um eine Impulsbreite 18 verlängert, so daß die Kurve 12 nach Durchlaufen mehrerer Perioden gegenüber der Kurve 13 eine Phasenverschiebung 20 aufweist, die sich im gezeichneten Beispiel jede zweite Periode stufenweise erhöht hat. Ein dem Frequenzteiler 2 nachgeschalteter weiterer Frequenzteiler 5 teilt die Frequenz weiter 1 : 2 zur Impulskurve 15. Ein Impulsbreitenregler 6 gibt einen in seiner Breite genau definierten Impuls 16 für jede Periode der Kurve 15 an die digitale Auswahleinrichtung 4 und sperrt damit die digitale Auswahleinrichtung 4 auf die Dauer der Impulsbreite der Kurve 16. Durch Regelung der Impulsbreite 16 kann daher die Anzahl der ausgesperrten Impulse und damit die Stufenhöhe der Phasenverschiebungszunahme verändert werden. Wird auf einmal eine Anzahl Impulse entsprechend der halben Periodendauer der Kurve 12 gesperrt, dann ist die Stufenhöhe der Phasenverschiebung 180 Grad. Durch Änderung des Übersetzungsverhältnisses des Teilers 5 kann außerdem die Stufenlänge variiert werden. 18 stellt die digitale Zunahme der Phasenverschiebung in jeder zweiten Periode dar. In der Kurvenform- und Verstärkerstufe 7 wird auch die Kurve 12 in eine Sinuskurve umgewandelt und gibt dann im Körper 9 zusammen mit der ebenfalls in eine Sinuskurve umgewandelten Kurve 13 die Interferenzhüllkurve 17.

Fig. 2

1 ist ein hochfrequenter Oszillator, aus dem mittels dem Teiler 20 niederfrequente Taktimpulse 11 gewonnen werden und durch den Teiler 3 in eine 4 KHz-Rechteckschwingung 13 umgewandelt werden. Durch die Kurvenform und Verstärkerstufe 7 wird diese Rechteckschwingung in eine Sinusschwingung umgewandelt.

Der Ausgang des Taktteilers 20 ist gleichzeitig über eine Additionsschaltung 21, in diesem Falle ein Odergatter, mit dem Frequenzteiler 2 verbunden. Die am Ausgang des Frequenzteilers 2 gewonnene Rechteckkurve 12 wird über eine Kurvenumwandlungs- und Verstärkerstufe 7 in eine Sinuskurve umgewandelt. Mittels der Additionsschaltung 21 wird über die Sperrschaltung 22, in diesem Falle ein Undgatter, höherfrequente Impulse, die direkt dem Oszillator über den Umschalter 27 vom Frequenzteiler 20 entnommen werden, zusätzlich zwischen den Taktimpulsen 11 dem Frequenzteiler 2 zugeführt. Anstelle des Frequenzteilers 20 und des Oszillators 1, sowie der Sperrschaltung 22 können in einer erfindungsgemäßen Variante auch zwei Oszillatoren mit unterschiedlicher Frequenz Verwendung finden, wobei der höherfrequente Oszillator als Start-Stopp-Oszillator ausgeführt ist. Die am Ausgang des Frequenzteilers 2 auftretende Kurve 12 wird über einen weiteren umschaltbaren Frequenzteiler 25 und einen weiteren Frequenzteiler 26 zur Steuerung eines Multiplexers 24 verwendet. Der Multiplexer 24 schaltet also im Takt des Ausgangs des Frequenzteilers 26 verschiedene Übersetzungsstufen des Frequenzteilers 25 auf einen Eingang des Undgatters 23, dessen zweiter Eingang direkt mit dem Ausgang des Frequenzteilers 2 verbunden ist. Hierdurch wird die Impulsbreite der Kurve 12 zur Steuerung der Öffnungszeit der Sperrvorrichtung 22 herangezogen, während die Auswahl der Perioden durch den Multiplexer 24 und den Frequenzteiler 25 erfolgt. Die abgegebene Impulsbreite des Frequenzteilers 25 darf hierbei nicht größer als die Periodenlänge der Rechteckkurve 12 sein. Die Funktion dieser Anordnung ist folgende:

Der Frequenzteiler 2 bewirkt, daß nur eine gewisse Anzahl von Impulsen, z. B. 12 Impulse, zur Bildung einer Ausgangsperiode benützt werden. Bei einer Taktfrequenz von 48 KHz entstehen daher am Ausgang des Teilers 2 4 KHz. Wird dem Frequenzteiler 2 während der halben Periode der Kurve 12 die doppelte Frequenz, also 96 KHz, zugeführt, dann verkürzt sich die halbe Periode um die Hälfte, so daß innerhalb dieser Periode eine Phasenverschiebung von 90 Grad entsteht. Man kann also die Größe der Phasenverschiebung innerhalb einer Periode durch die Höhe der zweiten Frequenz bestimmen. Es ist daher eine Umschalteinrichtung 27 vorgesehen, mit der am Frequenzteiler 20 verschiedene Teilerstufen für die Abnahme der höheren Frequenz benützt werden können. Verwendet man als hinzuzufügende höhere Frequenz z. B. eine 100fache Frequenz, also 4,8 MHz, dann ändert sich die Phasenverschiebung innerhalb einer Periode um annähernd 180 Grad, so daß als Interferenzhüllkurve 17 eine Rechteckkurve entsteht. Von der Anzahl der Perioden, in denen keine Phasenverschiebung durchgeführt wird, hängt die Breite dieser Rechteckkurve und damit die Frequenz dieser Interferenzhüllkurve 17 ab. Die Anzahl der gleichbleibenden Perioden bestimmt hierbei der Frequenzteiler 25 und der Multiplexer 24. Wird daher in einem wesentlich langsameren Rhythmus als die Interferenzkurve durch den Frequenzteiler 26 der Multiplexer 24 weitergeschaltet, so wird sich die Frequenz dieser Interferenzkurve periodisch ändern. Je nach Einstellung der Frequenzteiler 25 können Frequenzänderungen z. B. zwischen 0 und 10 oder 0 und 100 oder 90 und 100 Hz der Interferenzhüllkurven, wie sie zur Zeit in der Interferenzpraxis üblich sind, gewonnen werden.

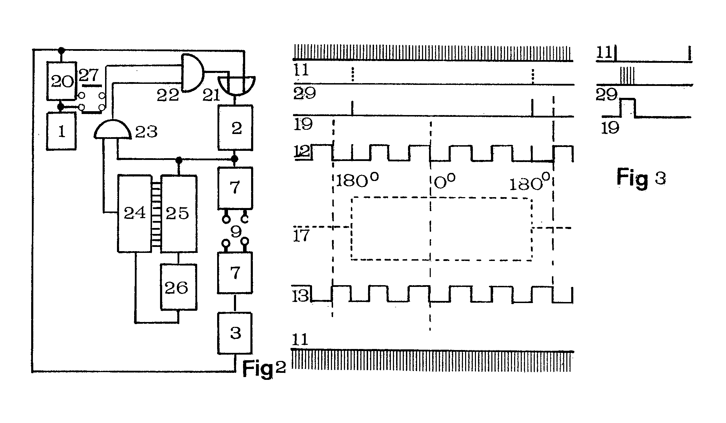

Fig. 3 zeigt die Kurven 11, 29 und 19 in einem vergrößerten Zeitmaßstab, wobei 11 die Taktfrequenz für den Frequenzteiler 2 ist, 29 die zugeschaltete höhere Frequenz und 19 der Steuerimpuls, der die Frequenzen zuführt, und am Ausgang von 23 auftritt.

**Patentansprüche**

1. Interferenzstromtherapiegerät mit einem oder mehreren Oszillatoren (1), mit dem zwei oder mehr niederfrequente Wechselströme (12, 13) gleicher Frequenz in Form von Impulsen erzeugt werden, bei dem eine Einrichtung (4) zur Beeinflussung der relativen Phasenverschiebung zwischen den niederfrequenten Wechselströmen vorhanden ist und bei dem die niederfrequenten Wechselströme in einem zu behandelnden Körper zur Interferenz gebracht werden, dadurch gekennzeichnet, daß dem bzw. den Oszillatoren (1) jeweils Frequenzteiler (2) nachgeschaltet sind, wobei zumindest an einem Frequenzteiler (2) eine Einrichtung in Form einer digitalen Auswahleinrichtung (4) angeschlossen ist, die wahlweise einen oder mehrere Impulse eines der beiden Wechselströme (14) fortläßt oder einen oder mehrere Impulse (29) hinzufügt oder das Teilerverhältnis des Frequenzteilers (2) ändert, wodurch die relative Phasenlage (20) der beiden Wechselströme (12, 13) zueinander verändert wird, und daß die digitale Auswahleinrichtung (4) mit einer Steuereinrichtung (26) verbunden ist, die die zeitliche Lage (18) der ausgewählten Perioden — in denen die Veränderungen stattfinden — festlegt und damit den Strom (12) in seiner Phasenlage verschiebt, wobei die Dauer der gleichbleibenden Phasenverschiebung die Breite der einzelnen Stufen und die Größe der Änderung die Stufenhöhe der stufenartigen Interferenzhüllkurve (17) bestimmt.

2. Interferenzstromtherapiegerät nach Anspruch 1 dadurch gekennzeichnet, daß zwischen Oszillator (1) und Frequenzteiler (2) als digitale Auswahleinrichtung (4) eine Takt-Sperrschaltung vorgesehen ist, die von der Steuerschaltung (5, 6) gesteuert wird und im Bereich der ausgewählten Ausgangsperiode eine vorgegebene Anzahl Taktimpulse sperrt.

3. Interferenzstromtherapiegerät nach Anspruch 1, dadurch gekennzeichnet, daß dem Frequenzteiler (2) ein weiterer Frequenzteiler (5) als Steuerschaltung nachgeschaltet ist, der einen Impulsbreitenregler (6) zur Veränderung der Ausgangsimpulsbreite besitzt.

4. Interferenzstromtherapiegerät nach Anspruch 1, dadurch gekennzeichnet, daß zwischen Oszillator (1) und Frequenzteiler (2) die digitale Auswahleinrichtung in Form einer Additionsschaltung (21), die mit ihrem Ausgang an den Frequenteiler (2), mit ihrem einen Eingang über einen Taktteiler (20) an den Oszillator (1) und mit ihrem zweiten Eingang über eine Sperrschaltung (22) mit einem höherfrequenten Ausgang des Taktteilers (20) oder umschaltbar direkt am Oszillator (1) angeschlossen ist, wobei der Schalteingang der Sperrschaltung (22) über ein Undgatter (23) mit der Steuerschaltung verbunden ist, so daß im Abstand der durch die Steuerschaltung bestimmten Zeit höherfrequente Taktimpulse den Frequenzteiler erreichen.

5. Interferenzstromtherapiegerät nach Anspruch 1, dadurch gekennzeichnet, daß die Steuerschaltung aus einem Multiplexer (24) und einem Frequenzteiler (25) besteht, wobei der Multiplexer (24) verschiedene Teilerstufen des Frequenzteilers (25) hintereinander an die digitale Auswahleinrichtung anschließt und der Schalteingang des Multiplexers (24) mit einem weiter untersetzten Ausgang des Teilers verbunden ist, der den Multiplexer (24) schrittweise weiterschaltet.

6. Interferenzstromtherapiegerät nach Anspruch 1, dadurch gekennzeichnet, daß der Oszillator (1), die Frequenzteiler (2, 3), die digitale Auswahleinrichtung (4) und die Steuereinrichtung aus einem Mikroprozessor bestehen.

**Claims**

1. Apparatus for interference current therapy, with one or several oscillators (1) producing two or several low-frequency alternating currents (12, 13) of identical frequency in form of pulses, and with a device (4) for influencing the relative phase shifts between said lowfrequency alternating currents and in which these lowfrequency alternating currents are brought to interference in a biological object to be treated, said apparatus being characterised by the fact that respective frequency dividers (2) are connected in series to the oscillator(s) (1), at least one frequency divider (2) being connected to a digital selector device (4) which either omits one or several pulses of one of the two alternating currents (14), or adds one or several pulses (29), or alters the division ratio of the frequency divider (2), thereby modifying the relative phase position (20) of the two alternating currents (12, 13) to each other, and by the fact that said digital selector device (4) is connected to a control divice (26) which determines the timing (18) of the periods selected in which alterations take place, thus shifting the phase position of the current (12), the duration of said phase shift determining the width of the individual steps and the extent of alteration in step height of the step-like interference envelipe (17).

2. Apparatus for interference current therapy according to claim 1, characterised by the fact that a digital selector device (4) in form of a phase blocking circuit is arranged between oscillator (1) and frequency divider (2), which is controlled by the control circuit (5, 6) and blocks a pre-set number of phase pulses within the output period selected.

3. Apparatus for interference current therapy according to claim 1, characterised by the fact that another frequency divider (5) is connected in series to the first frequency divider (2), which acts as a control circuit and has a pulse width controller (6) for modifying output pulse width.

4. Apparatus for interference current therapy according to claim 1, characterised by the fact that between oscillator (1) and frequency divider (2) there is a digital selector device in form of an adder (21), which is connected to the frequency divider (2) by its input, to the oscillator (1) by one of its outputs via a phase divider (20), and to a higher-Frequency output of said phase divider (20) by its second output via a blocking circuit (22), or which can be directly switched over to the oscillator (1), the input of the blocking circuit (22) being connected to the control circuit via an UND gate (23) so that higher-frequency phase pulses will reach the frequency divider (2) at intervalls determined by the control circuit.

5. Apparatus for interference current therapy according to claim 1, characterised by the fact that the control circuit consists of a mutliplexer (24) and a frequency divider (25), the multiplexer (24) subsequently connecting different steps of the frequency divider (25) to the digital selector device, and the input of the multiplexer (24) being connected to another lower output of the divider (25) for step-by-step switching of the multiplexer (24).

6. Apparatus for interferency current therapy according to claim 1, characterised by the fact that the oscillator (1), the frequency divider (2, 3), the digital selector divice (4) and the control device consist of a microprocessor.

## Revendications

1. Appareil de thérapie par courants interférentiels comportant un ou plusieurs oscillateurs (1), à l'aide desquels deux ou plusieurs courants alternatifs à basse fréquence identique (12, 13) sont produits sous la forme d'impulsions, dans lequel un dispositif (4) est prévu pour influencer le déphasage relatif entre les courants alternatifs à basse fréquence et dans lequel les courants alternatifs à basse fréquence sont amenés à interférer dans un corps devant être traité, caractérisé par le fait qu'en aval du ou des oscillateurs (1) sont branchés des diviseurs de fréquence (2) respectifs qu'au moins à un diviseur de fréquence (2) est raccordé un dispositif constitué sous la forme d'un dispositif numérique de sélection (4) qui délivre au choix une ou plusieurs impulsions de l'un des deux courants alternatifs (14) ou ajoute une ou plusieurs impulsions (29) ou modifie le rapport de division du diviseur de fréquence (2), avec comme effet une modification de la position de phase relative (20) des deux courants alternatifs (12, 13), et que le dispositif numérique de sélection (4) est relié à un dispositif de commande (26) qui fixe la position dans le temps (18) des périodes sélectionnées — dans lesquelles les modifications interviennent — et décalent par conséquent la position de phase du courant (12), la durée du déphasage constant déterminant la largeur des différents échelons et la valeur de la modification de la hauteur des échelons de la courbe enveloppe d'interférence (17) possédant une forme échelonnée.

2. Appareil de thérapie par courants interférentiels suivant la revendication 1, caractérisé par le fait qu'entre l'oscillateuer (1) et le diviseur de fréquence (2) est prévu, en tant que dispositif numérique de sélection (4), un circuit de blocage de cadence qui est commandé par le circuit de commande (5, 6) et bloque un nombre prédéterminé d'impulsions de cadence pendant l'intervalle de la période de sortie sélectionnée.

3. Appareil de thérapie par courants interférentiels suivant la revendication 1, caractérisé par le fait qu'en aval du diviseur de fréquence (2) est branché, en tant que circuit de commande, un autre diviseur de fréquence (5) qui possède un régulateur des durées d'impulsion (6) servant à modifier la durée des impulsions de sortie.

4. Appareil de thérapie par courants interférentiels suivant la revendication 1, caractérisé par le fait qu'entre l'oscillateur (1) et le diviseur de fréquence (2), le dispositif numérique de sélection possède la forme d'un circuit additionneur (21), dont la sortie est raccordée au diviseur de fréquence (2), dont une entrée est raccordée par l'intermédiaire d'un diviseur de cadence (20) à l'oscillateur (1) et dont la seconde entrée est reliée par l'intermédiaire d'un circuit de blocage (22) à une sortie à fréquence supérieure du diviseur de cadence (20) ou est raccordée d'une manière commutable directement à l'oscillateuer (1), l'entrée de commutation du circuit de blocage (22) étant reliée par l'intermédiaire d'une porte Et (23) du circuit de commande de sorte que des impulsions de cadence à fréquence supérieure atteignent le diviseur de fréquence avec un décalage dans le temps déterminé par le circuit de commande.

5. Appareil de thérapie par courants interférentiels suivant la revendication 1, caractérisé par le fait que le circuit de commande est constitué par un multiplexeur (24) et par un diviseur de fréquence (25), le multiplexeur (24) raccordant successivement différents échelons de division du diviseur de fréquence (25) au dispositif numérique de sélection, et l'entrée de commutation du multiplexeur (24) étant reliée à une sortie à taux de division supérieur du diviseur, qui fait avancer pas-à-pas le multiplexeur (24).

6. Appareil de thérapie par courant interférentiel suivant la revendication 1, caractérisé par le fait que l'oscillateur (1), les diviseurs de fréquence (2,3), le dispositif numérique de sélection (4) et le dispositif de commande sont constitués par un microprocesseur.

Fig 1

Fig 2

Fig 3